# EUROPÄISCHE PATENTSCHRIFT

(11) **EP 1 780 575 B1**
(45) Veröffentlichungstag und Bekanntmachung des Hinweises auf die Patenterteilung: **16.06.2010**
(21) Anmeldenummer: 06018926.3
(22) Anmeldetag: 10.09.2006
(51) Int. Cl.: G02B 23/24, G02B 3/14, A61B 1/00

(54) **Kamera**
Camera
Caméra

(30) Priorität: 27.10.2005 DE 102005051714
(43) Veröffentlichungstag der Anmeldung: 02.05.2007
(60) Teilanmeldung: 09175365.7
(73) Patentinhaber: LINOS Photonics GmbH & Co. KG, 37081 Göttingen (DE)
(72) Erfinder: Guse, Frank, 63454 Hanau (DE); Bürckner-Koydl, Dieter, 86415 Mering (DE); Partheymüller, Ulrich, 83607 Holzkirchen (DE); Knoch, Stefan, 81547 München (DE)
(74) Vertreter: Oberhardt, Knut

(56) Entgegenhaltungen:
- WO-A1-2004/077126
- DE-U1- 29 824 899
- FR-A1- 2 769 375
- FR-A1- 2 876 267
- US-A- 5 071 229
- US-A1- 2004 228 003
- US-B1- 6 288 767
- [Online] 3 March 2004 (2004-03-03), Retrieved from the Internet: URL:http://www.dpreview.com/news/0403/0403 0302philipsfluidlens.asp>

## Beschreibung

Die Erfindung betrifft eine gattungsgemässe Kamera mit einem Gehäuse, mit einem darin angeordneten, ein Objekt auf einen Bildabnehmer abbildenden Objektiv, mit einer dessen Fokussierung elektrisch ändernden Fokussiereinrichtung und mit einer Steuerung für diese.

Eine solche Kamera ist bekannt (DE 101 25 772 A1). Bei dieser bekannten Kamera wird ein von einer als an dem Gehäuse angebrachten Handhabe ausgebildeten Steuerung gesteuerter Elektromotor vorgesehen, dessen Achse mit einer Verstellspindel versehen ist. Diese ist in einem Stellglied geführt, an welchem der Bildabnehmer festgelegt ist und aufgrund der Drehung der Achse des Elektromotors verstellt werden kann, so dass durch die Fokussiereinrichtung die Brennweite des Objektivs durch die Relativlage von zumindest einer Linse zu den anderen Linsen des Objektivs oder dessen Relativlage zu dem Bildabnehmer geändert werden kann.

Von Nachteil bei dieser bekannten Kamera ist die Tatsache der Notwendigkeit sowohl des gesonderten Stellgliedes als auch der Verstellspindel, neben dem Elektromotor selbst, welche Teile zusätzlichen Bauraum im Inneren des Gehäuses benötigen. Hierdurch wird verhindert, dass die Kamera klein baut. Ausserdem wird dadurch das Design und die Ergonomie solcher Kameras negativ beeinflusst, wenngleich die Fokussierung damit in einfacher Weise zu bewerkstelligen ist.

Ferner ist eine gattungsgemäße Dentalkamera nach dem Oberbegriff des unabhängigen Anspruchs bekannt (DE 29824899 U1).

Als Fokussiereinrichtung wirkt eine verschiebbare Objektivbaugruppe. Die Abbildung erfolgt über ein Zwischenbild.

Darüber hinaus ist es bekannt Flüssiglinsen zur Fokussierung zu verwende (Philips Fluid Lenses, 3.3.2004, Digital Photography Review).

Der Erfindung liegt die Aufgabe zugrunde, eine gattungsgemäße Kamera so weiterzubilden, dass sie ohne bewegliche Bauteile fokussieren kann.

Diese Aufgabe wird bei einer gattungsgemässen Kamera gemäss dem Hauptanspruch gelösts

Erfindungsgemäss wird in Abweichung vom Stand der Technik anstelle der hierbei verwendeten, elektromotorisch verstellbaren Stellglieder etc. also erfindungsgemäss eine Fokussiereinrichtung mit einem elektrooptischen Bauelement, vorzugsweise mit einer variablen Flüssiglinse eingesetzt, die elektronisch durch die Steuerung so gesteuert wird, dass ihre Abbildungseigenschaften in Form der Fokussierung auf verschiedene Objektabstände geändert werden, ohne dass Teile des Objektivs bewegt oder die Relativlage zu dem Bildabnemer geändert werden müssen.

Mit der Erfindung wird also der Vorteil erzielt, dass in dem Bauraum der Kamera weder ein Elektromotor noch eine Drehspindel noch ein zusätzliches Stellglied, sondern nur eine ohnehin notwendige Linse als elektrooptisches Bauelement in dem Objektiv als variable Flüssiglinse vorgesehen werden muss, insoweit also kein zusätzlicher Bauraum erforderlich ist. Infolgedessen ergibt sich insbesondere bei kleinen Kameras wie Dentalkameras der Vorteil einer elektronischen Fokussierung, ohne die Grösse des Bauraums und die dadurch bedingte Ergonomie der Kamera negativ zu beeinflussen. Ausserdem erfolgt die Verstellung praktisch leistungslos, also ohne eine solche elektrische Verlustleistung, wie sie ein Elektromotor benötigt.

Es ist vorteilhaft, wenn die variable Flüssiglinse im Bereich unmittelbarer Nähe einer Aperturblende oder deren Bildes angeordnet ist, weil dann eine besonders kleine Bauform der Kamera erzielt werden kann.

In zweckmässiger Ausgestaltung der Erfindung kann in dem Objektiv eine verstellbare Blende vorgesehen sein, deren Durchmesser in einem vorgebbaren, bestimmten Verhältnis zur Fokuslage einstellbar ist. Durch die Fokussierung kann zugleich ein dementsprechendes Abblenden von einer kleinen Öffnung der Blende bei kleinem Aufnahmeabstand auf eine grosse Öffnung bei einem grossen Aufnahmeabstand mit dem einstellbaren Verhältnis von Durchmesser zur Fokuslage erfolgen. Der Antrieb wird dabei elektrisch und automatisch entsprechend der Fokuslage betätigt.

Entweder ist diese verstellbare Blende einerseits mit dem Gehäuse und andererseits mit einem Antrieb -oder umgekehrt- jeweils fest verbunden und z.B. als Irisblende ausgebildet.

Besonders einfach wird die Einstellung der verstellbaren Blende, wenn diese als Flüssigkristallblende (LCD) ausgebildet ist und -ebenfalls praktisch leistungslos- über eine weitere elektrische Spannung eingestellt wird. Ausserdem ergibt sich auch hier der Vorteil, dass keine Wärme durch elektrische Verlustleistung, wie bei einem Elektromotor erzeugt wird.

In bevorzugter Weiterbildung der Erfindung ist die Steuerung als Prozessor des Bildabnehmers zur Ermittlung der Bildschärfe ausgebildet, führt dessen Ausgang die elektrische Spannung für die variable Flüssiglinse führt und ist der Ausgang an die variable Flüssiglinse angeschlossen, weil dann manuell eine Einstellung des Fokus nicht erforderlich ist, vielmehr selbsttätig erfolgt. Dabei ist gar keine Handhabe zur Fokussierung mehr erforderlich, was die Möglichkeit der Reinigung der Kamera sowie Desinfektionierung bei der Ausbildung und Verwendung als Dentalkamera erheblich vereinfacht.

Weitere zweckmässige Ausgestaltung und Weiterbildungen der Erfindung sind in den weiteren Unteransprüchen gekennzeichnet.

Ein Ausführungsbeispiel der Erfindung wird nachfolgend unter Bezugnahme auf die Zeichnung näher erläutert, die die Kamera im einen schematischen Halbschnitt zeigt.

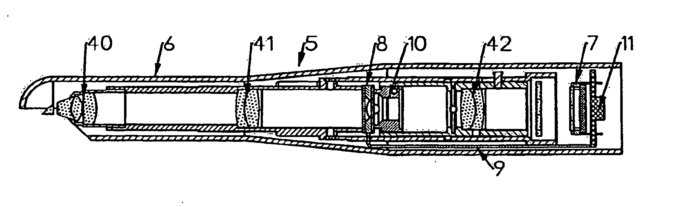

Die in Figur 1 insgesamt mit 5 bezeichnete Kamera weist ein Gehäuse 6 mit einem darin angeordneten Objektiv 40,41,42 mit Linsen 40,41,42 auf, welches ein Objekt auf einen Bildabnehmer 7 abbildet. Hierbei weist das Objektiv 40,41,42 ein ein zwischen dem Objekt und dem Bildabenehmer liegendes reelles Zwischenbild erzeugendes Teilobjektiv 40 auf und ist mit einer weiteren optischen Anordnung 41,42 versehen ist, welche dann das reelle Zwischenbild auf den Bildabnehmer 7 abbildet. Die weitere optische Anordnung 41,42 weist eine benachbart zu dem Teilobjektiv 40 angeordnete erste 41 und eine davon beabstandet angeordnete zweite Linsengruppe 42 auf.

In dem Gehäuse 6 ist eine als mittels einer elektrischen Spannung in ihren Abbildungseigenschaften veränderbare variable Flüssiglinse 8 -als Teil des Objektivs- ausgebildete, dessen Fokussierung elektrisch ändernde Fokussiereinrichtung vorgesehen, die von einer Steuerung gesteuert ist. Mit 9 ist die elektrische Zuführung zu der variablen Flüssiglinse 8 dargestellt. Die variable Flüssiglinse 8 ist hierbei zwischen der ersten 41 und der zweiten Linsengruppe 42 angeordnet.

Ferner weist das Gehäuse 6 der Kamera 5 in dem Objektiv eine verstellbare Blende 10 auf, deren Durchmesser in einem bestimmten Verhältnis zur Fokuslage einstellbar und als Flüssigkristallblende (LCD) ausgebildet ist, die über eine weitere elektrische Spannung gesteuert ist. Dieser Anschluss ist aus Gründen der Übersichtlichkeit in der Zeichnung nicht dargestellt.

Der Bildabnehmer 7 ist ferner mit der als Prozessor 11 zur Ermittlung der Bildschärfe ausgebildeten Steuerung versehen, dessen Ausgang die elektrische Spannung für die variable Flüssiglinse 8 führt und an deren Eingang angeschlossen ist. Diese Ausbildung der Kamera eignet sich insbesondere für den Einsatz als Dentalkamera.

## Patentansprüche

1. Kamera (5) mit einem Gehäuse (6), mit einem darin angeordneten, ein Objekt auf einen Bildabnehmer (7) abbildenden Objektiv,mit einer dessen Fokussierung elektrisch ändernden Fokussiereinrichtung und mit einer Steuerung für diese, wobei die Fokussiereinrichtung ein mittels einer elektrischen Spannung in seinen Abbildungseigenschaften veränderbares elektrooptisches Bauelement (8) aufweist, wobei die Kamera als Dentalkamera ausgebildet ist, dass das elektro-optische Bauelement als variable Flüssiglinse (8) ausgebildet ist, wobei die variable Flüssiglinse (8) Teil des Objektivs (40,41,42) ist,
wobei das Objektiv (40,41,42) ein zwischen dem Objekt und dem Bildabenehmer liegendes, ein reelles Zwischenbild erzeugendes Teilobjektiv (40) aufweist und mit einer weiteren optischen Anordnung (41,42) versehen ist, welche das reelle Zwischenbild auf den Bildabnehmer (7) abbildet,
wobei die variable Flüssiglinse (8) zwischen dem reellen Zwischenbild und dem Bildabnehmer (7) angeordnet ist,
wobei die weitere optische Anordnung (41,42) eine benachbart zu dem Teilobjektiv (40) angeordnete erste (41) und eine davon beabstandet angeordnete zweite Linsengruppen (42) aufweist und
wobei zwischen diesen Linsengruppen die variable Flüssiglinse (8) angeordnet ist.

2. Kamera nach Anspruch 1, **dadurch gekennzeichnet, dass** die variable Flüssiglinse (8) im Bereich unmittelbarer Nähe einer Aperturblende angeordnet ist.

3. Kamera nach Anspruch 1 oder 2, **dadurch gekennzeichnet, dass** die variable Flüssiglinse (8) im Bereich unmittelbarer Nähe des Bildes der Aperturblende angeordnet ist.

4. Kamera nach einem der Ansprüche 1 bis 3, **dadurch gekennzeichnet, dass** in dem Objektiv (40,41,42) eine verstellbare Blende (10) vorgesehen ist, deren Durchmesser in einem bestimmten Verhältnis zur Fokuslage einstellbar ist.

5. Kamera nach Anspruch 4, **dadurch gekennzeichnet, dass** die in dem Objektiv (40,41,42) vorgesehene Blende einerseits fest mit dem Gehäuse und andererseits fest mit einem Antrieb -oder umgekehrt- verbunden ist.

## Claims

1. A camera (5) comprising a housing (6), an objective lens for imaging an object onto an image sensor, a focusing means for electrically adjusting the focusing of the objective lens, and a controller for the focusing means,
wherein the focusing means includes an electro-optical component (8) adjustable in its imaging characteristics by means of an electric voltage,
wherein the camera is configured as a dental camera, the electro-optical component is configured as a variable liquid lens (8), wherein the variable liquid lens (8) is part of the objective lens (40, 41, 42),
wherein the objective lens (40, 41, 42) includes a partial objective (40), disposed between the object and the image sensor, for generating a real intermediate image, and provided with a further optical assembly (41, 42), which images the real intermediate image onto the image sensor (7),
wherein the variable liquid lens (8) is arranged between the real intermediate image and the image sensor (7),
wherein the further optical assembly (41, 42) includes a first lens group (41) arranged adjacent to the partial objective (40) and a second lens group (42) arranged at a distance thereto, and wherein the variable liquid lens (8) is arranged between these lens groups.

2. The camera according to claim 1, **characterised in that** the variable liquid lens (8) is arranged in the area in the immediate vicinity of an aperture diaphragm.

3. The camera according to claim 1 or 2, **characterised in that** the variable liquid lens (8) is arranged in the area in the immediate vicinity of the image of the aperture diaphragm.

4. The camera according to any one of claims 1 to 3, **characterised in that** an adjustable diaphragm (10) is provided in the objective lens (40, 41, 42) having a diameter adjustable in a specified ratio to the focal position.

5. The camera according to claim 4, **characterised in that** the diaphragm provided in the objective lens (40, 41, 42) is on the one hand fixedly connected to the housing and the on the other hand fixedly connected to a drive, or vice versa.

## Revendications

1. Appareil photographique (5) avec un boîtier (6), avec un objectif agencé à l'intérieur, reproduisant un objet sur un lecteur d'image (7), avec un dispositif de focalisation modifiant électriquement sa focalisation et avec une commande pour celui-ci, dans lequel le dispositif de focalisation présente un élément électro-optique (8) modifiable dans ses propriétés de reproduction à l'aide d'une tension électrique, dans lequel l'appareil photographique est conçu comme un appareil photographique dentaire, l'élément électro-optique est conçu comme une lentille liquide variable (8), dans lequel la lentille liquide variable (8) fait partie de l'objectif (40, 41, 42), dans lequel l'objectif (40, 41, 42) présente un objectif partiel (40) se situant entre l'objet et le lecteur d'image, produisant une image intermédiaire réelle, et est doté d'un autre dispositif optique (41, 42), qui reproduit l'image intermédiaire réelle sur le lecteur d'image (7), dans lequel la lentille liquide variable (8) est disposée entre l'image intermédiaire réelle et le lecteur d'image (7), dans lequel l'autre dispositif optique (41, 42) présente un premier groupe de lentilles (41) disposé de façon contiguë à l'objectif partiel (40) et un deuxième groupe de lentilles (42) disposé à une certaine distance de celui-ci et dans lequel la lentille liquide variable (8) est disposée entre ces groupes de lentilles.

2. Appareil photographique selon la revendication 1, **caractérisé en ce que** la lentille liquide variable (8) est disposée dans la zone à proximité immédiate d'un diaphragme d'ouverture.

3. Appareil photographique selon la revendication 1 ou 2, **caractérisé en ce que** la lentille liquide variable (8) est disposée dans la zone à proximité immédiate de l'image du diaphragme d'ouverture.

4. Appareil photographique selon l'une des revendications 1 à 3, **caractérisé en ce que** dans l'objectif (40, 41, 42) est prévu un diaphragme réglable (10) dont le diamètre est réglable dans une certaine proportion par rapport à la position d'un point focal.

5. Appareil photographique selon la revendication 4, **caractérisé en ce que** le diaphragme prévu dans l'objectif (40, 41, 42) est relié, d'une part, fixement au boîtier et, d'autre part, fixement à un dispositif d'entraînement - ou inversement.
